# EUROPÄISCHE PATENTSCHRIFT

(11) **EP 3 125 860 B1**
(45) Veröffentlichungstag und Bekanntmachung des Hinweises auf die Patenterteilung: **05.02.2020**
(21) Anmeldenummer: 15710811.9
(22) Anmeldetag: 20.03.2015
(51) Int. Cl.: A61Q 17/04, A61K 8/04, A61K 8/49, A61K 8/81

(54) **KOSMETISCHE ZUBEREITUNG ZUM UV-SCHUTZ AUF DER BASIS VON ACRYLAT-COPOLYMEREN**
COSMETIC PREPARATIONS FOR UV PROTECTION ON THE BASIS OF ACRYLATE COPOLYMERS
PRÉPARATIONS COSMÉTIQUES POUR LA PROTECTION ANTI UV SUR LA BASE DES COPOLYMÈRES D'ACRYLATE

(30) Priorität: 01.04.2014 DE 102014206221
(43) Veröffentlichungstag der Anmeldung: 08.02.2017
(73) Patentinhaber: Beiersdorf AG, 20253 Hamburg (DE)
(72) Erfinder: BLECKMANN, Andreas, 22926 Ahrensburg (DE); LERG, Heike, 22303 Hamburg (DE); SCHLENKER, David, 22607 Hamburg (DE)
(86) Internationale Anmeldenummer: PCT/EP2015/055931
(87) Internationale Veröffentlichungsnummer: WO 2015/150118

(56) Entgegenhaltungen:
- DE-A1-102007 028 497
- US-A1- 2006 159 649
- Anonymous: "Discover the latest ingredients, formulations and the most innovative beauty products", Innovation Zone 2012 In-cosmetics Asia, 1 November 2012 (2012-11-01), XP55206294,

## Beschreibung

Die vorliegende Erfindung betrifft eine kosmetische Zubereitung enthaltend ein Acrylat-Copolymer mit einer Glasübergangstemperatur von -5 bis -15 C°, gemessen mittels DSC und partikulären UV-Filtern.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Eine relativ neue Produktform zur Hautpflege stellen die unter der Dusche eingesetzten Hydrodispersionen dar, die nach einem ersten Duschvorgang auf die nasse Haut appliziert werden, wobei anschließend in einem zweiten Duschvorgang überschüssige, nicht auf der Haut fixierte Zubereitung abgespült wird (sogenannte In-shower Produkte, die analog wie Duschgele angewendet werden, im Gegensatz zu diesen jedoch nicht reinigen, sondern das Pflegeprodukt auf die Haut applizieren).

Nachteilig an den herkömmlichen In-Shower Produkten ist der Umstand, dass sich mit ihnen kaum UV-Lichtschutzfilter für den UV-Schutz der Haut wirksam applizieren lassen. Meist werden die UV-Filter beim zweiten Duschvorgang mehr oder weniger vollständig von der Haut wieder abgespült, so dass praktisch kein UV-Schutz entstehen kann.

Es war daher die Aufgabe der vorliegenden Erfindung, ein In-Shower Produkt zu entwickeln, mit dem sich größeren Mengen an UV-Filtern wirksam auf die Haut applizieren lassen. Insbesondere sollten Lichtschutzfaktoren von mindestens SPF 6 bei einer normalen Anwendung erreichen lassen.

Überraschend gelöst wird die Aufgabe durch eine kosmetische Zubereitung gemäß Anspruch 1.

Zwar kennt der Stand der Technik die DE 102007028497 sowie die Veröffentlichung "Discover the latest ingredients, forrmulations and the most innovative beauty products", Innovation Zone 2012 In-cosmetics Asia, vom 1.11.2012, doch konnten diese Schriften nicht den Weg zur vorliegenden Erfindung weisen.

US2006/0159649 A1 beschreibt mittels Acrylaten stabilisierte in-shower Produkte. Die Lösung der Aufgabe ist insbesondere deshalb überraschend, weil der Fachmann davon ausgehen musste, dass sich partikuläre UV-Filter bei der In-shower Anwendung eine besonders geringe Anhaftung an der Haut aufzeigen und beim zweiten Duschvorgang zur Agglomerat-Bildung (Mikroverklumpung) neigen.

Die erfindungsgemäße Glasübergangstemperatur wird dabei mit Hilfe der Differential Scanning Calorimetry bestimmt. Im Rahmen der vorliegenden Erfindung (d.h. erfindungsgemäß) wurde ein Mettler DCS 1 Gerät im Temperaturzyklus-Modus mit einem gelochten Aluminium-Tiegel (40µl), bei einer Messtemperatur von -50 °C bis +150 °C, einer Heizrate von 10 °C/min. und Stickstoff als Spülgas (Stickstoff 5.0) verwendet.

Die Hautbefeuchtung wird erfindungsgemäß mit dem folgenden Verfahren bestimmt: Zunächst wird in einer 1. Messung die Hautfeuchte mit dem Corneometer C 825 (Firma Courage & Khazaka) an 4 Arealen an den Unterarmen bestimmt. Dieser Wert entspricht der Hautfeuchte unbehandelter Haut.

Anschließend werden die vermessenen Areale an den Unterarmen wie folgt vorkonditioniert: Sie werden 10s unter laufendem Wasser, ca. 32-35°C warm, gehalten, dann mit 2mg/cm² Duschbad ,10s einschäumt und unter laufendem Wasser 15s abgespült.

Daraufhin werden die nasse Areale 10s mit der zu testenden Zubereitung in einer Auftragungsmenge von 2mg/cm² eincremt, 15s mit 32-35°C warmen Wasser abwaschen und mit einem Handtusch trocken getupft und die Hautfeuchte mit dem Corneometer C 825 gemessen. Die Messung wir nach 1h wiederholt.

Es ist erfindungsgemäß vorteilhaft, wenn die Zubereitung das Acrylat-Copolymer in einer Konzentration von 0,1 bis 10 Gewichts-%, bezogen auf das Gesamtgewicht der Zubereitung, enthält.

Es ist erfindungsgemäß bevorzugt, wenn die Zubereitung das Acrylat-Copolymer in einer Konzentration von 0,5 bis 5% Gewichts-%, bezogen auf das Gesamtgewicht der Zubereitung, enthält.

Erfindungsgemäß vorteilhafte Ausführungsformen der vorliegenden Erfindung sind dadurch gekennzeichnet, dass die Gesamtmenge an partikulären UV-Filtern in der Zubereitung 1 von bis 20 Gewichts- %, bezogen auf das Gesamtgewicht der Zubereitung, beträgt.

Es ist erfindungsgemäß vorteilhaft, wenn als partikuläre UV-Filter 2,2'-Methylen-bis-(6-(2H-benzotriazol-2-yl)-4-(1,1,3,3-tetramethylbutyl)-phenol); 2,4,6-Tribiphenyl-4-yl-1,3,5-triazin; Titandioxid und/oder Zinkoxid eingesetzt werden. Erfindungsgemäß bevorzugt ist 2,4,6-Tribiphenyl-4-yl-1,3,5-triazin und Zinkoxid.

Außerdem ist es erfindungsgemäß bevorzugt, Kombinationen aus mindestens zwei der UV-Filter 2,2'-Methylen-bis-(6-(2H-benzotriazol-2-yl)-4-(1,1,3,3-tetramethylbutyl)-phenol); 2,4,6-Tribiphenyl-4-yl-1,3,5-triazin; Titandioxid und Zinkoxid einzusetzen. In diesem Fall sind Kombinationen mit 2,2'-Methylen-bis-(6-(2H-benzotriazol-2-yl)-4-(1,1,3,3-tetramethylbutyl)-phenol) und/oder Titandioxid besonders bevorzugt.

Die bevorzugte Einsatzkonzentration für 2,4,6-Tribiphenyl-4-yl-1,3,5-triazin beträgt von 1 bis 10 Gewichts-%, bezogen auf das Gesamtgewicht der Zubereitung.

Die bevorzugte Einsatzkonzentration für Zinkoxid beträgt von 1 bis 20 Gewichts %, bezogen auf das Gesamtgewicht der Zubereitung.

Erfindungsgemäß vorteilhaft enthält die erfindungsgemäße Zubereitung Glycerin.

In einem solchen Falle sind erfindungsgemäß vorteilhafte Ausführungsformen der vorliegenden Erfindung dadurch gekennzeichnet, dass die Zubereitung Glycerin in einer Konzentration von 0,1 bis 20 Gewichts-%, bezogen auf das Gesamtgewicht der Zubereitung, enthält.

Das erfindungsgemäße Acrylat-Copolymer kann beispielsweise unter dem Handelsnamen Epitex 66 Polymer bei der Firma Dow erworben werden.

Es ist erfindungsgemäß vorteilhaft, wenn die Zubereitung einen oder mehrere UV-Filter enthält, die gewählt werden aus der Gruppe der Verbindungen 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; Terephthalidendicamphersulfonsäure; Verbindungen 2-Phenylbenzimidazol-5-sulfonsäure und/oder deren Salze und/oder Phenylen-1,4-bis-(2-benzimidazyl)-3,3'-5,5'-tetrasulfonsäuresalze; 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-Benzylidencampher; Ethylhexylsalicylat; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)-ester; 4-Methoxyzimtsäure(2-ethylhexyl)ester; 4-Methoxyzimtsäureisoamylester; 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); Merocyanine.

Es ist erfindungsgemäß bevorzugt, wenn als UV-Filter eine oder mehrere Verbindungen gewählt aus der Gruppe 4-(tert.-Butyl)-4'-methoxydibenzoylmethan, Ethylhexylsalicylat, Homomenthylsalicylat, 2-Ethylhexyl-2-cyano-3,3-diphenylacrylat, 4,4',4"-(1,3,5-Triazin-2,4,6-triyltriimino)-tris-benzoesäure-tris(2-ethylhexylester) und 2,4-Bis-{[4-(2-ethyl-hexyloxy)-2-hydroxy]-phenyl}-6-(4-methoxyphenyl)-1,3,5-triazin gewählt werden.

Erfindungsgemäße Ausführungsformen sind außerdem dadurch gekennzeichnet, dass die Zubereitung frei ist von Propyl- und Butylparaben, 3-lod-2-propinylbutylcarbamat, Methylisothiazolinon, 3-(4-Methylbenzyliden)-campher und 2-Hydroxy-4-methoxybenzophenon (Oxybenzon).

Hingegen kommt man zu erfindungsgemäß vorteilhaften Ausführungsformen, wenn die Zubereitung Ethylhexylglycerin, Propylenglycol, Butylenglycol, 2-Methylpropan-1,3-diol, 1,2-Pentandiol, 1,2-Hexandiol, 1,2-Octandiol und/oder 1,2-Decandiol enthält.

Außerdem ist es erfindungsgemäß vorteilhaft, wenn die Zubereitung Phenoxyethanol und/oder Methylparaben enthält.

Es ist vorteilhaft im Sinne der vorliegenden Erfindung, wenn die Zubereitung einen oder mehrere Wirkstoffe gewählt aus der Gruppe der Verbindungen Magnolienextrakt, Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, ß-Alanin und/oder Licochalcon A, enthält.

Erfindungsgemäß vorteilhaft ist es auch, wenn die erfindungsgemäße Zubereitung einen oder mehrere Komplexbildner enthält. Erfindungsgemäß bevorzugt ist dabei der Einsatz von Ethylendiamintetraessigsäure-Alkalisalzen (EDTA).

Erfindungsgemäß bevorzugt enthält die erfindungsgemäße Zubereitung von 0,02 bis 1,5% Gewichts-% EDTA, bezogen auf das Gesamtgewicht der Zubereitung.

Erfindungsgemäß vorteilhaft ist es auch, wenn die Zubereitung einen oder mehrere Emulgatoren gewählt aus der Gruppe der Verbindungen Glycerylstearatcitrat, Cetearylalkohol, Natriumcetearylsulfat + Glycerylstearat, Cetearylsulfosuccinat Natriumstearoylglutamat, Polyglyceryl-3-methylglucosedistearat, Stearinsäure, Kaliumcetylphosphat, enthält. Dabei ist ein Gehalt von Polyglyceryl-3-methylglucosedistearat erfindungsgemäß bevorzugt.

Enthält die erfindungsgemäße Zubereitung Polyglyceryl-3-methylglucosedistearat, so wird dieser Emulgator in einer Konzentration von 0,05 bis 5 Gewichts-%, bezogen auf das Gesamtgewicht der Zubereitung eingesetzt.

Es ist erfindungsgemäß von Vorteil, wenn die erfindungsgemäße Zubereitung Hydroxyethylcellulose und/oder Acrylat/C10-30 Alkylacrylat Crosspolymer enthält.

Erfindungsgemäß vorteilhafte Ausführungsformen der vorliegenden Erfindung sind dadurch gekennzeichnet, dass die Zubereitung Hydroxyethylcellulose in einer Konzentration von 0,05 bis 5 Gewichts-%, bezogen auf das Gesamtgewicht der Zubereitung, enthält.

Enthält die Zubereitung Acrylat/C10-30 Alkylacrylat Crosspolymer, so ist es erfindungsgemäß bevorzugt, wenn diese Verbindung in einer Konzentration von 0,05 bis 2,5 Gewichts-%, bezogen auf das Gesamtgewicht der Zubereitung eingesetzt wird.

Erfindungsgemäß vorteilhafte Ausführungsformen sind auch dadurch gekennzeichnet, dass die Zubereitung Ethanol enthält.

Enthält die Zubereitung Ethanol, so ist es erfindungsgemäß bevorzugt, wenn diese Verbindung in einer Konzentration von 1 bis 15 Gewichts-%, bezogen auf das Gesamtgewicht der Zubereitung eingesetzt wird.

Die Ölphase der erfindungsgemäßen Zubereitung kann darüber hinaus noch weitere Öl-, Fett- und Wachskomponenten enthalten, beispielsweise polaren Öle aus der Gruppe der Lecithine oder Verbindungen wie z. B. Cocoglycerid, Capryl/Caprinsäure Triglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr. Auch Verbindungen wie Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat können eingesetzt werden.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid gewählt werden. Es ist insbesondere vorteilhaft, wenn die Ölphase der erfindungsgemäßen Formulierungen einen Gehalt an C₁₂₋₁₅-Alkylbenzoat aufweist.

Vorteilhafte Ölkomponenten sind ferner z. B. Isopropylpalmitat, Myristylmyristat, Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Erfindungsgemäß bevorzugt ist es, wenn die erfindungsgemäße Zubereitung Dialkylcarbonat, Dialkyladipat und/oder Dialkylglutarat enthält.

Erfindungsgemäß besonders bevorzugt ist es, wenn die erfindungsgemäße Zubereitung Din-Octylcarbonat (INCI Dicaprylyl Carbonate) und/oder Di-n-Butyladipat (INCI Dibutyl Adipate) enthält.

Die erfindungsgemäße Zubereitung kann darüber hinaus die üblichen Inhaltstoffe enthalten und wie eine übliche Zubereitung zusammengesetzt sein.

Die erfindungsgemäße Zubereitung kann vorteilhaft als Tagespflegeprodukt oder Sonnenschutzmittel eingesetzt werden.

Besonders vorteilhaft ist es, die erfindungsgemäße Zubereitung als In-shower Produkt einzusetzen. Daher sind In-shower Produkte auf der Basis einer erfindungsgemäßen kosmetischen Zubereitung erfindungsgemäß.

Beschrieben wird auch die Verwendung der erfindungsgemäßen kosmetischen Zubereitung als In-shower Produkt.

Beschrieben wird die Verwendung dieser erfindungsgemäßen Zubereitung zum Schutz der Haut vor UV-Strahlung.

Beschrieben wird das Verfahren zum Schutz der Haut vor UV-Strahlung, welches dadurch gekennzeichnet ist, dass eine erfindungsgemäße Zubereitung, die einen oder mehrere der erfindungsgemäß vorteilhaften UV-Filter enthält, nach dem Duschen auf die nasse Haut aufgetragen wird und anschließend überschüssige Reste der Zubereitung mittels einer Wasserdusche wieder abgespült werden.

Vorteilhaft schließt sich bei Verfahren als weiterer Verfahrensschritt das Abtrocknen der Haut (bevorzugt mittels eines Handtuches) an.

### Vergleichsversuch

Mit dem folgenden Versuch konnte der erfindungsgemäße Effekt beispielhaft belegt werden: Es wurden die folgenden Rezepturen hergestellt:

| | **1** | **2** | **3** |
|---|---|---|---|
| **INCI** | **m [%]** | **m [%]** | **m [%]** |
| Epitex 66 Acrylates Copolymer¹ | 2,00 | | |
| Vergleichspolymer Acrylates Copolymer² | | 2,00 | |
| Polyglyceryl-3 Methylglucose Distearate | 0,10 | 0,10 | 0,10 |
| Parfum | 0,40 | 0,40 | 0,40 |
| Glycerin + Aqua | 1,00 | 1,00 | 1,00 |
| Aqua + Sodium Hydroxide | 0,30 | 0,30 | 0,30 |
| Phenoxyethanol | 0,50 | 0,50 | 0,50 |
| Hydroxyethylcellulose | 0,10 | 0,10 | 0,10 |
| Acrylates/C10-30 Alkyl Acrylate Crosspolymer | 0,20 | 0,20 | 0,20 |
| Carbomer | 0,10 | 0,10 | 0,10 |
| Aqua | 52,27 | 50,47 | 56,27 |
| Alcohol Denat. + Aqua | 10,00 | 10,00 | 10,00 |
| Trisodium EDTA | 1,00 | 1,00 | 1,00 |
| Butyl Methoxydibenzoylmethane | 3,50 | 3,50 | 3,50 |
| Phenylbenzimidazole Sulfonic Acid | 0,10 | 0,10 | 0,10 |
| Ethylhexyl Salicylate | 4,50 | 4,50 | 4,50 |
| Ethylhexyl Triazone | 1,00 | 1,00 | 1,00 |
| Octocrylene | 9,00 | 9,00 | 9,00 |
| Homosalate | 9,00 | 9,00 | 9,00 |
| Bis-Ethylhexyloxyphenol Methoxyphenyl Triazine | 1,00 | 1,00 | 1,00 |
| Diethylhexyl Butamido Triazone | 0,50 | 0,50 | 0,50 |
| Diethylamino Hydroxybenzoyl Hexyl Benzoate | 1,00 | 1,00 | 1,00 |
| Methylene Bis-Benzotriazolyl Tetramethylbutylphenol | 3,00 | 3,00 | 3,00 |
| Zinc Oxid | 1,00 | 1,00 | 1,00 |

| | | | |
|---|---|---|---|
| ¹Einsatzmenge Epitex 66=4,4 Gew-%. Diese entspricht dann der angegebenen Acrylates Copolymer Konzentration von 2 Gewichts-% (effektiv Wert) ²Daitosol 3000SLPN von Daitosakai mit einer Glasübergangstemperatur von + 14,5 | | | |

Von diesen Zubereitungen wurde die Hautbefeuchtungsleistung mit dem folgenden Verfahren bestimmt:
Zunächst wird in einer 1. Messung die Hautfeuchte mit dem Corneometer C 825 (Firma Courage & Khazaka) an 4 Arealen an den Unterarmen bestimmt. Dieser Wert entspricht der Hautfeuchte unbehandelter Haut.

Anschließend werden die vermessenen Areale an den Unterarmen wie folgt vorkonditioniert: Sie werden 10s unter laufendem Wasser, ca. 32-35°C warm, gehalten, dann mit 2mg/cm² Duschbad, 10s einschäumt und unter laufendem Wasser 15s abgespült.

Daraufhin werden die nasse Areale 10s mit der zu testenden Zubereitung in einer Auftragungsmenge von 2mg/cm2 eincremt, 15s mit 32-35°C warmen Wasser abwaschen und mit einem Handtusch trocken getupft und die Hautfeuchte mit dem Corneometer C 825 gemessen. Die Messung wir nach 1h wiederholt.

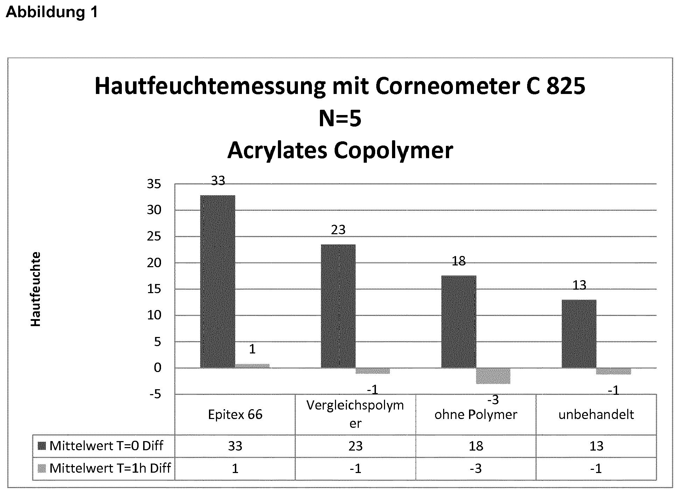

Die Ergebnisse des Vergleichsversuches sind in Abbildung 1 zu finden

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | **4** | **5** | **6** |
|---|---|---|---|
| **INCI** | **m [%]** | **m [%]** | **m [%]** |
| Epitex 66 (Acrylates Copolymer)¹ | 2,0 | 2,0 | 2,0 |
| Polyglyceryl-3 Methylglucose Distearate | 0,1 | 0,1 | 0,1 |
| Parfum | 0,4 | 0,4 | 0,4 |
| Glycerin + Aqua | 5,0 | 5,0 | 4,6 |
| Aqua + Sodium Hydroxide | 0,3 | 0,3 | 0,3 |
| Phenoxyethanol | 0,5 | 0,5 | 0,5 |
| Hydroxyethylcellulose | 0,1 | 0,1 | 0,1 |
| Acrylates/C10-30 Alkyl Acrylate Crosspolymer | 0,2 | 0,2 | 0,2 |
| Carbomer | 0,1 | 0,1 | 0,1 |
| Alcohol Denat. + Aqua | 10,0 | 10,0 | 10,0 |
| Trisodium EDTA | 1,0 | 1,0 | 1,0 |
| Butyl Methoxydibenzoylmethane | 3,5 | 4,3 | 4,9 |
| Ethylhexyl Salicylate | | | 4,5 |
| Ethylhexyl Triazone | 1,0 | | 1,0 |
| Octocrylene | 9,0 | 5,0 | 9,0 |
| Homosalate | | 9,0 | 9,0 |
| Bis-Ethylhexyloxyphenol Methoxyphenyl Triazine | 1,0 | 1,0 | 1,0 |
| Diethylhexyl Butamido Triazone | 0,5 | 0,5 | 0,5 |
| Diethylamino Hydroxybenzoyl Hexyl Benzoate | 1,0 | 1,0 | 1,0 |
| Phenylbenzimidazole Sulfonic Acid | 2,0 | | |
| Disodium Phenyl Dibenzimidazole Tetrasulfonate | 0,5 | | |
| Methylene Bis-Benzotriazolyl Tetramethylbutylphenol | 2,0 | 3,0 | 4,4 |
| Tris-Biphenyl Triazine | 2,0 | | 3,0 |
| Titanium Dioxide | 1,0 | 1 | 3,2 |
| Zinc Oxide | 4,00 | 5,0 | 1,00 |
| Aqua | ad 100 | ad 100 | ad 100 |

| | | | |
|---|---|---|---|
| ¹Einsatzmenge Epitex 66=4,4 Gew-%. Diese entspricht dann der angegebenen Acrylates Copolymer Konzentration von 2 Gewichts-% (effektiv Wert) | | | |

## Patentansprüche

1. Kosmetische Zubereitung enthaltend
a) ein Acrylat-Copolymer mit einer Glasübergangstemperatur von -5 bis -15 C°, gemessen mittels DSC und
b) partikuläre UV-Filter,
wobei die Zubereitung frei ist von Propyl- und Butylparaben, 3-lod-2-propinylbutylcarbamat, Methylisothiazolinon, 3-(4-Methylbenzyliden)-campher und 2-Hydroxy-4-methoxybenzophenon (Oxybenzon).

2. Kosmetische Zubereitung nach Anspruch 1, **dadurch gekennzeichnet, dass** die Zubereitung das Acrylat-Copolymer in einer Konzentration von 0,5 bis 5 Gewichts-%, bezogen auf das Gesamtgewicht der Zubereitung, enthält.

3. Kosmetische Zubereitung nach einem der vorhergehenden Ansprüche, **dadurch gekennzeichnet, dass** als partikuläre UV-Filter 2,2'-Methylen-bis-(6-(2H-benzotriazol-2-yl)-4-(1,1,3,3-tetramethylbutyl)-phenol); 2,4,6-Tribiphenyl-4-yl-1,3,5-triazin; Titandioxid und/oder Zinkoxid eingesetzt werden.

4. Kosmetische Zubereitung nach einem der vorhergehenden Ansprüche, **dadurch gekennzeichnet, dass** als partikuläre UV-Filter Kombinationen aus mindestens zwei der UV-Filter 2,2'-Methylen-bis-(6-(2H-benzotriazol-2-yl)-4-(1,1,3,3-tetramethylbutyl)-phenol); 2,4,6-Tribiphenyl-4-yl-1,3,5-triazin; Titandioxid und Zinkoxid eingesetzt werden.

5. Kosmetische Zubereitung nach einem der vorhergehenden Ansprüche, **dadurch gekennzeichnet, dass** die Gesamtmenge an partikulären UV-Filtern in der Zubereitung von 1 bis Gewichts-20 %, bezogen auf das Gesamtgewicht der Zubereitung, beträgt.

6. Kosmetische Zubereitung nach einem der vorhergehenden Ansprüche, **dadurch gekennzeichnet, dass** die Zubereitung einen oder mehrere UV-Filter enthält, die gewählt werden aus der Gruppe der Verbindungen 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; Terephthalidendicamphersulfonsäure; Verbindungen 2-Phenylbenzimidazol-5-sulfonsäure und/oder deren Salze und/oder Phenylen-1,4-bis-(2-benzimidazyl)-3,3'-5,5'-tetrasulfonsäuresalze; 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-Benzylidencampher; Ethylhexylsalicylat; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); Merocyanine.

7. Kosmetische Zubereitung nach einem der vorhergehenden Ansprüche, **dadurch gekennzeichnet, dass** die Zubereitung Ethylhexylglycerin, Propylenglycol, Butylenglycol, 2-Methylpropan-1,3-diol, 1,2-Pentandiol, 1,2-Hexandiol, 1,2-Octandiol und/oder 1,2-Decandiol enthält.

8. Kosmetische Zubereitung nach einem der vorhergehenden Ansprüche, **dadurch gekennzeichnet, dass** die Zubereitung einen oder mehrere Wirkstoffe gewählt aus der Gruppe der Verbindungen Magnolienextrakt, Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, ß-Alanin und/oder Licochalcon A, enthält.

9. Kosmetische Zubereitung nach einem der vorhergehenden Ansprüche, **dadurch gekennzeichnet, dass** die Zubereitung Phenoxyethanol und/oder Methylparaben enthält.

10. Kosmetische Zubereitung nach einem der vorhergehenden Ansprüche, **dadurch gekennzeichnet, dass** die Zubereitung einen oder mehrere Komplexbildner enthält.

11. Kosmetische Zubereitung nach einem der vorhergehenden Ansprüche, **dadurch gekennzeichnet, dass** die Zubereitung einen oder mehrere Emulgatoren gewählt aus der Gruppe der Verbindungen Glycerylstearatcitrat, Cetearylalkohol Natriumcetearylsulfat + Glycerylstearat, Cetearylsulfosuccinat Natriumstearoylglutamat, Polyglyceryl-3-methylglucosedistearat,
Stearinsäure, Kaliumcetylphosphat, enthält.

12. Kosmetische Zubereitung nach einem der vorhergehenden Ansprüche, **dadurch gekennzeichnet, dass** die Zubereitung Hydroxyethylcellulose und/oder Acrylat/C10-30 Alkylacrylat Crosspolymer enthält.

13. Kosmetische Zubereitung nach einem der vorhergehenden Ansprüche, **dadurch gekennzeichnet, dass** die Zubereitung Ethanol enthält.

## Claims

1. Cosmetic preparation comprising
a) an acrylate copolymer having a glass transition temperature of -5 to - 15°C, measured by means of DSC, and
b) particulate UV filters,
wherein the preparation is free of propyl- and butylparaben, 3-iodo-2-propynyl butylcarbamate, methylisothiazolinone, 3-(4-methylbenzylidene)camphor and 2-hydroxy-4-methoxybenzophenone (oxybenzone).

2. Cosmetic preparation according to Claim 1, **characterized in that** the preparation contains the acrylate copolymer in a concentration of 0.5% to 5% by weight, based on the total weight of the preparation.

3. Cosmetic preparation according to either of the preceding claims, **characterized in that** the particulate UV filters used are 2,2'-methylenebis-(6-(2H-benzotriazol-2-yl)-4-(1,1,3,3-tetramethyl-butyl)phenol); 2,4,6-tribiphenyl-4-yl-1,3,5-triazine; titanium dioxide and/or zinc oxide.

4. Cosmetic preparation according to any of the preceding claims, **characterized in that** the particulate UV filters used are combinations of at least two of the UV filters 2,2'-methylenebis-(6-(2H-benzotriazol-2-yl)-4-(1,1,3,3-tetramethylbutyl)-phenol); 2,4,6-tribiphenyl-4-yl-1,3,5-triazine; titanium dioxide and zinc oxide.

5. Cosmetic preparation according to any of the preceding claims, **characterized in that** the total amount of particulate UV filters in the preparation is from 1% to 20% by weight, based on the total weight of the preparation.

6. Cosmetic preparation according to any of the preceding claims, **characterized in that** the preparation contains one or more UV filters selected from the group of compounds 1,4-di(2-oxo-10-sulfo-3-bornylidenemethyl)benzene and salts thereof; 4-(2-oxo-3-bornylidenemethyl)benzenesulfonic salts; 2-methyl-5-(2-oxo-3-bornylidenemethyl)sulfonic salts; terephthalidenedicamphorsulfonic acid; compounds of 2-phenylbenzimidazole-5-sulfonic acid and/or salts thereof and/or phenylene-1,4-bis(2-benzimidazoyl)-3,3';5,5'-tetrasulfonic salts; 2-(2H-benzotriazol-2-yl)-4-methyl-6-[2-methyl-3-[1,3,3,3-tetramethyl-1-[(trimethylsilyl)oxy]disiloxanyl]propyl]phenol; 3-benzylidenecamphor; ethylhexyl salicylate; 2-ethylhexyl 2-cyano-3,3-diphenylacrylate; 2-ethylhexyl 4-(dimethylamino)benzoate; amyl 4-(dimethylamino)benzoate; di(2-ethylhexyl) 4- methoxybenzalmalonate; 2-ethylhexyl 4-methoxycinnamate; isoamyl 4-methoxycinnamate; 2-hydroxy-4-methoxy-4'-methylbenzophenone; 2,2'-dihydroxy-4-methoxybenzophenone; homomenthyl salicylate; 2-ethylhexyl 2-hydroxybenzoate; dimethicodiethyl benzalmalonate; 3-(4-(2,2-bis(ethoxycarbonylvinyl)phenoxy)propenyl)methoxy-siloxane/dimethylsiloxane copolymer; 4-(tert-butyl)-4'-methoxydibenzoylmethane; hexyl 2-(4'-diethylamino-2'-hydroxybenzoyl)benzoate; dioctylbutylamidotriazone (INCI: Diethylhexyl-Butamidotriazone); 2,4-bis[5-1(dimethylpropyl)benzoxazol-2-yl(4-phenyl)imino]-6- (2-ethylhexyl)imino-1,3,5-triazine with (CAS No. 288254-16-0); 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); merocyanine.

7. Cosmetic preparation according to any of the preceding claims, **characterized in that** the preparation contains ethylhexylglycerol, propylene glycol, butylene glycol, 2-methylpropane-1,3-diol, pentane-1,2-diol, hexane-1,2-diol, octane-1,2-diol and/or decane-1,2-diol.

8. Cosmetic preparation according to any of the preceding claims, **characterized in that** the preparation contains one or more active ingredients selected from the group of compounds magnolia extract, glycyrrhetic acid, urea, arctiin, alpha-lipoic acid, folic acid, phytoene, D-biotin, coenzyme Q10, alpha-glucosylrutin, carnitine, carnosine, caffeine, natural and/or synthetic isoflavonoids, glycerylglucose, creatine, creatinine, taurine, tocopherol, tocopherol acetate, β-alanine and/or licochalcone A.

9. Cosmetic preparation according to any of the preceding claims, **characterized in that** the preparation contains phenoxyethanol and/or methyl-paraben.

10. Cosmetic preparation according to any of the preceding claims, **characterized in that** the preparation contains one or more complexing agents.

11. Cosmetic preparation according to any of the preceding claims, **characterized in that** the preparation contains one or more emulsifiers selected from the group of compounds glyceryl stearate citrate, cetearyl alcohol, sodium cetearyl sulfate + glyceryl stearate, cetearyl sulfosuccinate, sodium stearoyl glutamate, polyglyceryl-3-methylglucose distearate, stearic acid, potassium cetyl phosphate.

12. Cosmetic preparation according to any of the preceding claims, **characterized in that** the preparation contains hydroxyethyl cellulose and/or Acrylate/C10-30 Alkyl acrylate crosspolymer.

13. Cosmetic preparation according to any of the preceding claims, **characterized in that** the preparation contains ethanol.

## Revendications

1. Préparation cosmétique contenant
a) un copolymère d'acrylate présentant une température de transition vitreuse de -5 à -15°C, mesurée par DSC, et
b) des filtres UV particulaires
la préparation étant exempte de propylparabène ainsi que de butylparabène, de butylcarbamate de 3-iodo-2-propynyle, de méthylisothiazolinone, de 3-(4-méthylbenzylidène)-camphre et de 2-hydroxy-4-méthoxybenzophénone (oxybenzone).

2. Préparation cosmétique selon la revendication 1, **caractérisée en ce que** la préparation contient le copolymère d'acrylate en une concentration de 0,5 à 5% en poids par rapport au poids total de la préparation.

3. Préparation cosmétique selon l'une quelconque des revendications précédentes, **caractérisée en ce qu'**on utilise, comme filtre UV particulaire, du 2,2'-méthylène-bis-(6-(2H-benzotriazol-2-yl)-4-(1,1,3,3-tétraméthylbutyl)-phénol) ; de la 2,4,6-tribiphényl-4-yl-1,3,5-triazine ; du dioxyde de titane et/ou de l'oxyde de zinc.

4. Préparation cosmétique selon l'une quelconque des revendications précédentes, **caractérisée en ce qu'**on utilise, comme filtre UV particulaire, des combinaisons d'au moins deux des filtres UV 2,2'-méthylène-bis-(6-(2H-benzotriazol-2-yl)-4-(1,1,3,3-tétraméthylbutyl)-phénol); 2,4,6-tribiphényl-4-yl-1,3,5-triazine ; dioxyde de titane et oxyde de zinc.

5. Préparation cosmétique selon l'une quelconque des revendications précédentes, **caractérisée en ce que** la quantité totale de filtres UV particulaires dans la préparation représente 1 à 20% en poids, par rapport au poids total de la préparation.

6. Préparation cosmétique selon l'une quelconque des revendications précédentes, **caractérisée en ce que** la préparation contient un ou plusieurs filtres UV, qui sont choisis dans le groupe des composés 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; acide téréphtalidènedicamphresulfonique ; acide 2-phénylbenzimidazole-5-sulfonique et/ou ses sels et/ou sels de l'acide phénylène-1,4-bis-(2-benzimidazyl)-3,3'-5,5'-tétrasulfonique ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-benzylidènecamphre ; salicylate d'éthylhexyle ; 2-cyano-3,3-diphénylacrylate de 2-éthylhexyle ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxy-4'-méthylbenzophénone ; 2,2'-dihydroxy-4-méthoxybenzophénone ; salicylate d'homomenthyle ; 2-hydroxybenzoate de 2-éthylhexyle ; diméthicodiéthylbenzalmalonate ; copolymère de 3-(4-(2,2-bis-éthoxycarbonylvinyl)-phénoxy)propényl)-méthoxysiloxane/diméthylsiloxane; 4-(tert-butyl)-4'-méthoxydibenzoylméthane ; ester hexylique de l'acide 2-(4'-diéthylamino-2'-hydoxybenzoyl)-benzoïque ; dioctylbutylamidotriazone (INCI Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine (présentant le n° CAS 288254-16-0) ; 2,4-bis-{[4-(2-éthylhexyloxy)-2-hydroxy]-phényl}-6-(4-méthoxyphényl)-1,3,5-triazine (INCI Bis-Ethylhexyloxyphenol Methoxyphenyl Triazin) ; ester tris(2-éthylhexylique) de l'acide 4,4',4"-(1,3,5-triazine-2,4,6-triyltriimino)-trisbenzoïque ester 2-éthylhexylique de l'acide 4,4',4"-(1,3,5-triazine-2,4,6-triyltriimino)-trisbenzoïque (également 2,4,6-tris-[anilino-(p-carbo-2'-éthyl-1'-hexyloxy)]-1,3,5-triazine (INCI Ethylhexyl Triazone) ; mérocyanines.

7. Préparation cosmétique selon l'une quelconque des revendications précédentes, **caractérisée en ce que** la préparation contient de l'éthylhexylglycérol, du propylèneglycol, du butylèneglycol, du 2-méthylpropane-1,3-diol, du 1,2-pentanediol, du 1,2-hexanediol, du 1,2-octanediol et/ou du 1,2-décanediol.

8. Préparation cosmétique selon l'une quelconque des revendications précédentes, **caractérisée en ce que** la préparation contient une ou plusieurs substances actives choisies dans le groupe des composés extrait de magnolia, acide glycyrrhétinique, urée, arctiine, acide alpha-liponique, acide folique, phytoène, D-biotine, coenzyme Q10, alpha-glucosylrutine, carnitine, carnosine, caféine, isoflavonoïdes naturels et/ou synthétiques, glycérylglucose, créatine, créatinine, taurine, tocophérol, acétate de tocophérol, β-alanine et/ou licochalcone A.

9. Préparation cosmétique selon l'une quelconque des revendications précédentes, **caractérisée en ce que** la préparation contient du phénoxyéthanol et/ou du méthylparabène.

10. Préparation cosmétique selon l'une quelconque des revendications précédentes, **caractérisée en ce que** la préparation contient un ou plusieurs complexants.

11. Préparation cosmétique selon l'une quelconque des revendications précédentes, **caractérisée en ce que** la préparation contient un ou plusieurs émulsifiants, choisis dans le groupe des composés citrate de stéarate de glycéryle, alcool cétéarylique, cétéarylsulfate de sodium + stéarate de glycéryle, sulfosuccinate de cétéaryle, stéaroylglutamate de sodium, distéarate de polyglycéryl-3-méthylglucose, acide stéarique, cétylphosphate de potassium.

12. Préparation cosmétique selon l'une quelconque des revendications précédentes, **caractérisée en ce que** la préparation contient de l'hydroxyéthylcellulose et/ou un copolymère réticulé d'acrylate/acrylate de C10-30-alkyle.

13. Préparation cosmétique selon l'une quelconque des revendications précédentes, **caractérisée en ce que** la préparation contient de l'éthanol.
